# EUROPÄISCHE PATENTSCHRIFT

(11) **EP 0 434 801 B1**
(45) Veröffentlichungstag und Bekanntmachung des Hinweises auf die Patenterteilung: **08.03.1995**
(21) Anmeldenummer: 90910090.1
(22) Anmeldetag: 17.07.1990
(51) Int. Cl.: C07D 307/62

(54) **VERFAHREN ZUR HERSTELLUNG VON DIKALIUM-ASCORBAT-2-SULFAT**
PROCESS FOR PRODUCING DIPOTASSIUM ASCORBATE-2-SULPHATE
PROCEDE DE FABRICATION DU 2-SULFATE D'ASCORBATE DE DIPOTASSIUM

(30) Priorität: 17.07.1989 CH 2673/89
(43) Veröffentlichungstag der Anmeldung: 03.07.1991
(73) Patentinhaber: ENCO ENGINEERING CHUR AG, CH-7000 Chur (CH)
(72) Erfinder: GUBEROVIC, Zeljko, YU-43300 Koprivnica (YU); HOHNJEC, Marijan, YU-41000 Zagreb (YU); KUFTINEC, Josip, YU-41421 Hrvatski/Leskovak (YU); OKLOBDZIJA, Milan, YU-41000 Zagreb (YU)
(74) Vertreter: Lauer, Joachim, Dr.
(86) Internationale Anmeldenummer: CH9000175
(87) Internationale Veröffentlichungsnummer: WO9101313

(56) Entgegenhaltungen:
- US-A- 3 954 809
- Handbook of Chem. and Physics, 52nd Ed. 1971 C-104
- Journal of the Chemical Society, Perkin Transactions 1, 1974 (London, GB), P.A. Seib et al; pages 1220-1124
- Chemical Abstracts, Vol. 87, No. 10, 5 September 1977, (Columbus; 0hio, US), see page 338, abstract 73386m,

## Beschreibung

### Technisches Gebiet

Die vorliegende Erfindung betrifft ein Verfahren zur Herstellung von Dikalium-Ascorbat-2-Sulfat der Formel 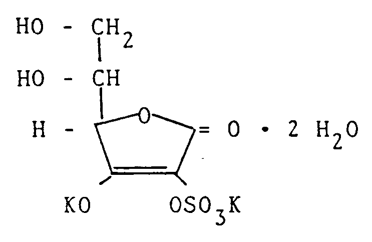
gemäss dem Oberbegriff des Patentanspruches 1.

Dikalium-Ascorbat-2-Sulfat (bzw. das entsprechende Dihydrat) ist ein Vitamin-C Derivat welches jedoch stabiler als Vitamin-C selbst ist. Es wird unter anderem verwendet als Zusatz zu Fischfutter (A.K.Soliman, K.Jauncey, R.J.Roberts; Aquaculture 60, 73, (1987)). Es ist ein Stoffwechselprodukt von Vitamin-C und wird in Fischen als Vitamin-C Reserve gebildet. Ein Mangel an Vitamin-C führt zur Mangelerkrankung Skorbut. Fische wie z.B. Lachse besitzen das zur Abspaltung des Sulfatrestes erforderliche Enzym (Sulfatase), wodurch ihr Organismus das reine Vitamin aus dem gespeicherten Derivat zurückbilden kann.

### Stand der Technik

Aus P.A. Seib et al., J. Chem. Soc. Perkin I, 1220, (1974), ist es bekannt, in Position 2 sulfatierte Ascorbinsäure durch die Reaktion von in den Positionen 5 und 6 geschützter Ascorbinsäure mit Pyridin-Schwefe-Trioxid-Komplex in einer Dimethyl-Formamid -Lösung herzustellen.

Aus der US-A 3,954,809 ist es bekannt, L-Ascorbat-2-Sulfat auch aus ungeschützter Ascorbinsäure bzw. deren Salzen über die Reaktion mit TrimethylaminSchwefel-Trioxid-Komplex in wässriger Lösung und in Gegenwart einer ausreichenden Menge einer freien Base, insbesondere von Natriumhydroxid, bei einem pH-Wert von 10 - 10,5 herzustellen. Durch die Gegenwart der freien Base wird die negative Ladung vom Sauerstoff-Atom in Position 3 zum Sauerstoff-Atom in Position 2 verschoben, wodurch dieses reaktiver und selektiver sulfatiert wird. Um die Vollständigkeit der Sulfatierungsreaktion zu erreichen, wird der Trimethylamin-Schwefel-Trioxid-Komplex generell im Ueberschuss zugegeben, wobei jedoch darauf hingewiesen wird, dass die Reaktion grundsätzlich auch mit im wesentlichen equimolaren Mengen möglich ist. Durch Hydrolyse des Schwefel-Komplex-Ueberschusses entstehen anorganische Sulfate. Aus Reinheitsgründen können diese nicht im Endprodukt belassen werden. Gleiches gilt für das bei der Reaktion entstandene und in Lösung befindliche Trimethylamin sowie noch vorhandenen nicht umgesetzten Komplex. Die Abtrennung des Trimethylamins erfolgt bei dem bekannten Verfahren durch Erhöhung des pH-Wertes auf etwa 12,5 und nachfolgendes Verdampfen oder durch Leiten des Reaktionsgemisches durch eine Säule mit einem Kationen-Austauscher-Harz. Die Abtrennung der anorganischen Sulfate erfolgt duch Zugabe von Barium-Hydroxid als Bariumsulfat. Nach Zugabe von Methanol und Filtrieren erhält man schliesslich Barium-Ascorbat-2-Sulfat in kristalliner Form. Dieses Bariumsalz ist für Fische jedoch toxisch. Im Hinblick auf eine Verwendung in Fischfutter müsste es erst noch in das weit besser geeignete Kaliumsalz überführt werden. Dies ist prinzipiell möglich durch Zugabe von K₂SO₄ zu einer Lösung des erhaltenen Beriumsalzes in Wasser, durch Abtrennung des sich bildenden Bariumsalzes mittels Filtration und dem anschliessenden Verdampfen des Filtrats. Eine andere durch B.M. Tolbert et al. in ANN. N.Y. ACAD. SCI./1975/, 258, 48-69 beschriebene Möglichkeit das Barium-Ascorbat-2-Sulfat in das korrespondierende Dikaliumsalz umzuwandeln, besteht darin, eine wässrige Lösung des Barium-Ascorbat-2-Sulfats durch eine DOWEX 50-K⁺ enthaltende Säule zu leiten.

Nach dem vorstehend beschriebenen Verfahren lässt sich zwar Dikalium-Ascorbat-2-Sulfat-Dihydrat in sehr hoher Reinheit herstellen. Insbesondere im Hinblick auf die sehr aufwendige Reinigung des Endproduktes wäre die Herstellung dieses Produktes für die Verwendung als Zusatz von Fischfutter im grosstechnischen Massstab nach dem bekannten Verfahren jedoch nicht sinnvoll, weil viel zu unwirtschaftlich.

Bei der bei dem bekannten Verfahren darüberhinaus erforderlichen portionsweisen Zugabe der Schwefelreagenz ist zudem eine Kontamination der Umgebung mit Trimethylamin nur schwer zu vermeiden.

Aus dem Handbook of Chemistry and Physics, 52^{nd} Edition, 1971, C-104 ist ebenfalls ein hier einschlägiges Verfahren bekannt. Es wird mit einem hohen Komplexüberschuss sowie mit einer verdünnten Ascorbat-Lösung gearbeitet. Nach Konzentration der Reaktionsmischung durch Verdampfen unter Vakuum, unter anderem zur Separierung von nicht umgesetztem Komplex, wird das gewünschte Endprodukt durch Zugabe von Methanol auskristallisiert.

### Darstellung der Erfindung

Es ist insbesondere Aufgabe der vorliegenden Erfindung, ein Verfahren zur Herstellung von für Fischfutter ausreichend reinem Dikalium-Ascorbat-2-Sulfat-Dihydrat anzugeben, welches im grosstechnischen Rahmen wirtschaftlich mit hoher Ausbeute ausführbar ist. Darüberhinaus soll eine Kontamination der Umgebung mit Trimethylamin-Schwefel-Trioxid-Komplex vermeidbar sein.

Die genannten sowie weitere Aufgaben werden gemäss der vorliegenden Erfindung gelöst durch ein Verfahren, wie es im Patentanspruch 1 gekennzeichnet ist. Vorteilhafte Ausgestaltungen der vorliegenden Erfindung sind in den abhängigen Patentansprüchen gekennzeichnet.

Bei dem erfindungsgemässen Verfahren wird demnach Trimethylamin-Schwefel-Trioxid-Komplex in nur geringem molaren Ueberschuss (< 8%) zu einer konzentrierten Lösung von Kalium-L-Ascorbat in Wasser (500 - 750 g/l) zugegeben und der pH-Wert dieser Reaktionsmischung durch sukkzessive Zugabe von Kaliumhydroxid auf einen Wert im Bereich zwischen 9,5 und 10,5 eingestellt und für die Dauer der Reaktion auf diesem Wert gehalten.

Durch Verwendung einer konzentrierten Kalium-Ascorbat-Lösung ergibt sich der wesentliche Vorteil, dass das an sich in Wasser gut lösliche Trimethylamin bereits während der Reaktion entweicht. Es bleibt also nicht wie bei dem bekannten Verfahren in Lösung und muss deshalb auch nicht aufwendig abgetrennt werden. Durch die hohe Konzentration ergibt sich darüberhinaus eine hohe Herstellungskapazität und optimale Ausnutzung der verwendeten Anlage.

Bedingt durch den nur geringen molaren Ueberschuss, in dem der Trimethylamin-Schwefel-Trioxid-Komplex zu der konzentrierten Lösung von Kalium-L-Ascorbat zugegeben wird, verbleibt am Ende der Reaktionszeit praktisch kein nicht umgesetzter Komplex mehr in der Reaktionsmischung. Auch können sich anorganische Sulfate nicht in grosser Menge parallel bilden. Nachdem das Trimethylamin bereits entwichen ist, kann das gewünschte Endprodukt direkt durch einfache Zugabe eines Alkanols, insbesondere von Ethanol, auskristallisiert werden. Der grösste Teil der anorganischen Sulfate bleibt dabei sogar in Lösung. Im Endprodukt lassen sich allenfalls noch 1 - 2 % der anorganischen Sulfate nachweisen, was im Hinblick auf die beabsichtigte Verwendung des Endproduktes als Zusatz zu Fischfutter nicht weiter störend ist. Damit entfällt bei dem erfindungsgemässen Verfahren im Vergleich zu dem vorstehend beschriebenen bekannten Verfahren die gesonderte Abtrennung des Trimethylamins, die Abtrennung nicht umgesetzten Komplexes, die Abtrennung der anorganischen Sulfate und schliesslich auch noch die Umwandlung von dem toxischen Bariumsalz zu dem gewünschten Kaliumsalz. Letzteres ist bedingt durch die Verwendung von Kalium-L-Ascorbat in wässriger Lösung als Ausgangsstoff und durch die Einstellung des pH-Wertes im basischen Bereich mittels Kaliumhydroxid.

Die Einstellung des pH-Wertes auf einen Wert von 10 für die Dauer der Reaktion erfolgt bei der vorliegenden Erfindung aus dem gleichen Grund wie bei dem vorbekannten Verfahren, um das Sauerstoff-Atom in Position 2 selektiv zu sulfatieren.

Der molare Ueberschuss, in dem der Trimethylamin-Schwefel-Trioxid-Komplex zu der wässrigen Kalium-Ascorbat-Lösung zugegeben wird, sollte vorzugsweise nur 6 % betragen.

Die Temperatur des Reaktionsgemisches wird vorzugsweise auf einen Wert im Bereich zwischen 57°C und 65°C eingestellt und weiter vorzugsweise während der Dauer der Reaktion von dem genannten unteren Wert zu dem genannten oberen Wert linear erhöht, letzteres im Hinblick auf die nachstehend noch erwähnte Rückgewinnung des entweichenden Trimethylamins.

Die gesamte Menge Trimethylamin-Schwefel-Trioxid-Komplex kann zu der wässrigen Kalium-Ascorbat-Lösung gleich zu Anfang der Reaktion zugegeben wird.

Anstatt von Kalium-L Ascorbat auszugehen, kann die konzentrierte Lösung von Kalium-Ascorbat in Wasser mit Vorteil auch durch Vermischen von L-Ascorbinsäure mit Wasser und Neutralisieren dieser Mischung mit Kaliumhydroxid hergestellt werden. Zum Schutz der Ascorbinsäure sollte die Temperatur dabei auf einem Wert unter 25°C gehalten werden.

Wie vorstehend schon erwänhnt, kann das während der Reaktion aus dem Reaktionsgemisch entweichende Trimethylamin zurückgewonnen werden. Es wird dazu vorzugsweise in einem chlorierten organischen Lösungsmittel, insbesondere in 1,2-Dichlorethan, Chloroform, Tetrachlorkohlenstoff oder Methylenchlorid (Dichlormethan), absorbiert. Dies kann in einer mit dem verwendeten Reaktionsgefäss verbundenen Absorptionsäule erfolgen, in welcher das Absorptionsmittel gut gekühlt zirkuliert. Durch die Kühlung sollte die Temperatur des Absorptionsmittles unterhalb von etwa 0° C gehalten werden. Die Einstellung der Temperatur der Reaktionsmischung im Reaktionsgefäss auf zunächst nur 57° C und deren anschliessende Erhöhung auf 65° C ist deshalb von Vorteil, weil dadurch eine zu starke, die Kapazität der Absorptionskolonne ggf. übersteigende Entwicklung von Trimethylamin zu Beginn der Reaktion vermieden wird.

Bei Verwendung der vorstehend erwähnten Absorptions- bzw. Lösungsmittel für das entweichende Trimethylamin ist es möglich, aus der erhaltenen Lösung den für das vorliegende Verfahren als Reagenz erforderlichen Trimethylamin-Schwefel-Trioxid-Komplex wieder herzustellen. Ein hierzu besonders geeignetes, ebenfalls im grosstechnischen Massstab wirtschaftlich ausführbares Verfahren besteht darin, zu der das Trimethylamin enthaltenden Lösung Chlorsulfonsäure zuzugeben und gasförmigen Ammoniak durch dieses Reaktionsgemisch zwecks Rückgewinnung von Amin aus zuvor als Nebenprodukt entstandenem Amin-Hydrochlorid zu leiten. Die Rückgewinnung des Amins mittels Ammoniak ist möglich, weil Amin-Hydrochlorid eine bessere Löslichkeit aufweist als Ammoniumchlorid. Das rückgewonnene Amin reagiert mit noch vorhandener Chlorsulfon-Säure zu dem gewünschten R₃N:SO₃-Komplex. Das entstandene Ammoniumchlorid kann durch Zugabe von Wasser und Rühren gelöst werden. Der gewünschte tertiäre Amin-Schwefel-Trioxid-Komplex kann schliesslich durch Filtrieren abgetrennt, gewaschen und nach Trocknung als trockenes Endprodukt erhalten werden. Während der Reaktion wird die Temperatur vorzugsweise auf einen Wert zwischen 0° und 30 C eingestellt. Es wird zumindest solange Ammoniak in das genannte Reaktionsgemisch geleitet, bis dessen pH-Wert alkalisch ist.

### Wege zur Ausführung der Erfindung

Nachfolgend wird die Erfindung anhand einiger Ausführungsbeispiele näher erläutert.

### Beispiel 1

Zu einer Mischung von 500 g (2,84 Mol) Ascorbinsäure und 300 ml Wasser wird ein erster Teil einer Lösung von 339 g (6 Mol) Kaliumhydroxid in 530 ml Wasser unter Rühren und Kühlen auf eine Temperatur unter 25° C zugegeben bis ein p_{H}-Wert von 8,5 erreicht ist. Danach werden 420 g Trimethylamin-Schwefel-Trioxid-Komplex (3 Mol) zu der entstandenen Kalium-Ascorbat-Lösung zugegeben. In einem Absorber-System, welches mit dem die vorgenannte Lösung enthaltenden Reaktionsgefäss verbundenen ist, werden 2000 ml Dichlormethan auf -10° C gekühlt zirkuliert. Es wird darauf geachtet, dass die Temperatur im Absorber nicht über etwa 0° C ansteigt. Nun wird die Temperatur im Reaktionsgefäss auf 57° C erhöht. Der pH-Wert wird durch Zugabe eines weiteren Teils der Kalium-Hydroxid-Lösung auf einen Wert von 10 eingestellt. Die im folgenden ablaufende Sulfatierungsreaktion benötigt ungefähr zwei Stunden. Während dieser Zeit wird der pH-Wert durch Zugabe des restlichen Teils der Kalium-Hydroxid-Lösung je nach Bedarf auf dem vorgenannten Wert gehalten und die Temperatur langsam auf 65° C erhöht. Die Reaktion ist vollständig, wenn die gesamte angesetzte Kalium-Hydroxid-Lösung zugegeben worden ist.

Die Reaktionsmischung im Reaktionsgefäss wird nun auf 15° C gekühlt. Dann werden 2300 ml Ethanol (96%) für eine Dauer von 3 Stunden zugegeben. Gleichzeitig wird die Temperatur auf 5° C abgesenkt. Die sich durch das Auskristallisieren des gewünschten Endproduktes bildende Suspension wird noch über eine weitere Stunde gerührt, bevor sie filtriert wird. Der das gewünschte Endprodukt in kristalliner Form enthaltende Filterrückstand wird noch mit 1400 ml Ethanol (70 %) gewaschen.

Bei einem Testversuch konnten auf die vorbeschriebene Weise 960 g trockenes Dikalium-Ascorbat-2-Sulfat als weisses Pulver mit einem Schmelzpunkt von 150 - 152° C entsprechend einer Ausbeute von (92 %) erhalten werden. Die Reinheit des Produktes konnte zu 98,6 % (HPLC-Analyse) bestimmt werden.

In dem gleichen Testversuch konnten in dem im Absorbersystem zirkulierenden Dichlormethan nach der Reaktion 153 g absorbiertes Trimethylamin nachgewiesen werden, entsprechend 85 % des threoretischen Wertes.

Nachfolgend wird ein Beispiel dafür angegeben, wie aus einer Lösung von Trimethylamin in Dichlormethan in einem gesonderten Verfahren Trimethylamin-Schwefel-Trioxid-Komplex zurückgewonnen werden kann:
33,5 ml (0,507 Mol) Chlorsulfonsäure werden langsam zu einer eisgekühlten und gut gerührten Lösung von 28,5 g (0,5 Mol) Trimethylamin in 150 ml Dichlormethan zugegeben. Die Temperatur wird auf einem Wert unterhalb von 20°C gehalten. Die erste Hälfte der Chlorsulfonsäure reagiert sehr exotherm and sollte daher sehr langsam zugegeben werden, wohingegen die zweite Hälfte weniger Aufmerksamkeit benötigt. Nachdem die gesamte Menge der Chlorsulfonsäure zugegeben worden ist, wird gasförmiger Ammoniak durch die Lösung geleitet bis ein basischer p_{H}-Wert erreicht ist. Zu der entstandenen Suspension wird unter kräftigem Rühren 80 ml Wasser zugegeben. Nach 15 Minuten wird das feste Produkt abfiltriert, dreimal mit jeweils 25 ml Wasser gewaschen und schliesslich getrocknet.

Auf die beschriebene Weise sollten 55,5 - 66,5 g, entsprechend einer Ausbeute von 80 - 94 %, trockenes Endprodukt (Trimethylamin-Schwefel-Trioxid-Komplex) herstellbar sein.

### Beispiel 2

1500 ml der Reaktionsmischung entsprechend Beispiel 1 werden nach Ende der Reaktion (ausgeführt ebenfalls entsprechend Beispiel 1) auf 0° C abgekühlt. Es werden Impfkristalle von reinem Dikalium-Ascorbat-2-Sulfat zugegeben und die Mischung über drei Stunden gerührt. Die Kristalle, welche sich während dieser Zeit gebildet haben, werden durch Filtration abgetrennt, mit 500 ml Ethanol (70 %) gewaschen und bei 60° C unter reduziertem Druck getrocknet.

In einem Testversuch konnten auf diese Weise 640 g (61 %) trockenes Endprodukt mit einer Reinheit von 99,83 % (HPLC-Analyse) erhalten werden.

Anschliessend wird die Mutter-Flüssigkeit unter reduziertem Druck konzentriert auf 400 ml und die Temperatur auf 5° C abgesenkt. Es werden 400 ml Ethanol (96 %) sukkzessive über eine Dauer von 2 Stunden zugegeben unter konstantem Rühren.

In dem genannten Testversuch konnten dadurch weitere 310 g (30 %) des gewünschten Endproduktes mit einer Reinheit von 97,8 % (HPLC-Analyse) erhalten werden.

## Patentansprüche

1. Verfahren zur Herstellung von Dikalium-Ascorbat- 2-Sulfat-Dihydrat der Formel 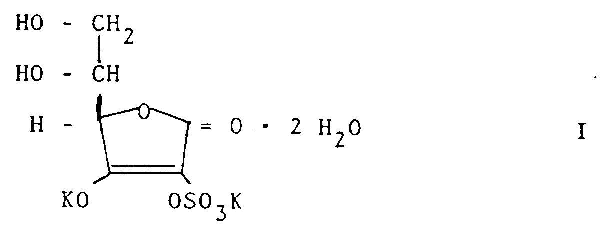 bei welchem Trimethylamin-Schwefel-Trioxid-Komplex in molarem Überschuss zu einer Lösung von Kalium-Ascorbat in Wasser zugegeben, der pH-Wert dieser Reaktionsmischung auf einen Wert im Bereich zwischen 9,5 und 10,5 eingestellt und für die Dauer der Reaktion auf diesem Wert gehalten und das während der Reaktion entstandene Dikalium-Ascorbat-2-Sulfat-Dihydrat durch Zugabe eines Alkanols isoliert wird, dadurch gekennzeichnet, dass der pH-Wert der Reaktionsmischung durch sukkzessive Zugabe von Kaliumhydroxid eingestellt wird, dass die Konzentration der Lösung von Kalium-Ascorbat in Wasser auf einen Wert im Bereich zwischen 500 und 750 g/l eingestellt wird, dass der molare Überschuss, in dem der Trimethylamin-Schwefel-Trioxid-Komplex zu der wässrigen Kalium-Ascorbat-Lösung zugegeben wird, nicht mehr als 8% beträgt und dass das während der Reaktion entstandene Dikalium-Ascorbat-2-Sulfat-Dihydrat direkt durch Zugabe des Alkanols zu der Reaktionsmischung aus dieser isoliert wird.

2. Verfahren nach Anspruch 1, dadurch gekennzeichnet, dass der pH-Wert auf einen Wert von 10 eingestellt und für die Dauer der Reaktion auf diesem Wert gehalten wird.

3. Verfahren nach einem der Ansprüche 1 oder 2, dadurch gekennzeichnet, dass der molare Ueberschuss, in dem der Trimethylamin-Schwefel-Trioxid-Komplex zu der wässrigen Kalium-Ascorbat-Lösung zugegeben wird, nicht mehr als 6% beträgt.

4. Verfahren nach einem der Ansprüche 1 bis 3, dadurch gekennzeichnet, dass die Temperatur des Reaktionsgemisches auf einen Wert im Bereich zwischen 57°C und 65°C eingestellt und vorzugsweise während der Dauer der Reaktion von dem genannten unteren Wert zu dem genannten oberen Wert linear erhöht wird.

5. Verfahren nach einem der Ansprüche 1 bis 4, dadurch gekennzeichnet, dass die gesamte Menge Trimethylamin-Schwefel-Trioxid-Komplex zu der wässrigen Kalium-Ascorbat-Lösung gleichzeitig zuzgegeben.

6. Verfahren nach einem Ansprüche 1 bis 5, dadurch gekennzeichnet, dass die konzentrierte Lösung von Kalium-Ascorbat in Wasser durch Vermischen von L-Ascorbinsäure mit Wasser und Neutralisieren dieser Mischung mit Kaliumhydroxid hergestellt wird.

7. Verfahren nach Anspruch 6, dadurch gekennzeichnet, dass die Temperatur bei der Herstellung der kontzentrierten Kalium-Ascorbat-Lösung auf einem Wert unter 25°C gehalten wird.

8. Verfahren nach einem der Ansprüche 1 bis 7, dadurch gekennzeichnet, dass das während der Reaktion entstandene Dikalium-Ascorbat-2-Sulfat-Dihydrat durch Zugabe von Methanol oder Ethanol, insbesondere von Ethanol, zu der Reaktionsmischung aus dieser isoliert wird.

9. Verfahren nach einem der Ansprüche 1 bis 8, dadurch gekennzeichnet, dass das während der Reaktion aus dem Reaktionsgemisch entweichende Trimethylamin in einem chlorierten organischen Lösungsmittel, insbesondere in 1,2-Dichlorethan, Chlorform, Tetrachlorkohlenstoff oder Mehtylenchlorid, absorbiert wird.

10. Verfahren nach Anspruch 9, dadurch gekennzeichnet, dass aus dem in dem chlorierten organischen Lösungsmittel absorbierten bzw. gelösten Trimethylamin tertiärer Amin-Schwefel-Trioxid-Komplex zurückgewonnen wird, indem Chlorsulfonsäure zu der Lösung zugegeben und gasförmiger Amoniak in das so erhaltene Reaktionsgemisch zwecks Rückgewinnung von Amin aus zuvor als Nebenprodukt entstandenem Amin-Hydrochlorid geleitet wird.

11. Verfahren nach Anspruch 10, dadurch gekennzeichnet, dass zumindest solange Ammoniak in das genannte Reaktionsgemisch geleitet wird, bis dessen pH-Wert alkalisch ist.

## Claims

1. Process for the preparation of dipotassium ascorbate 2-sulphate dihydrate of the formula 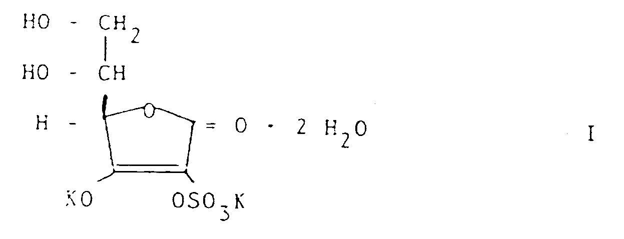 in which a trimethylamine-sulphur trioxide complex is added in molar excess to a solution of potassium ascorbate in water, the pH of this reaction mixture is brought to a value in the range between 9.5 and 10.5 and is kept at this value for the duration of the reaction, and the dipotassium ascorbate 2-sulphate dihydrate formed during the reaction is isolated by addition of an alkanol, characterized in that the pH of the reaction mixture is established by successive addition of potassium hydroxide, in that the concentration of the solution of potassium ascorbate in water is brought to a value in the range between 500 and 750 g/l, in that the molar excess in which the trimethylamine-sulphur trioxide complex is added to the aqueous potassium ascorbate solution is not more than 8 %, and in that the dipotassium ascorbate 2-sulphate dihydrate formed during the reaction is isolated directly from the reaction mixture by addition of the alkanol to this.

2. Process according to Claim 1, characterized in that the pH is brought to a value of 10 and is kept at this value for the duration of the reaction.

3. Process according to one of Claims 1 and 2, characterized in that the molar excess in which the trimethylamine-sulphur trioxide complex is added to the aqueous potassium ascorbate solution is not more than 6 %.

4. Process according to one of Claims 1 to 3, characterized in that the temperature of the reaction mixture is brought to a value in the range between 57°C and 65°C and is preferably increased linearly from the lower value mentioned to the upper value mentioned throughout the duration of the reaction.

5. Process according to one of Claims 1 to 4, characterized in that the entire amount of the trimethylamine-sulphur trioxide complex is added simultaneously to the aqueous potassium ascorbate solution.

6. Process according to one of Claims 1 to 5, characterized in that the concentrated solution of potassium ascorbate in water is prepared by mixing L-ascorbic acid with water and neutralizing this mixture with potassium hydroxide.

7. Process according to Claim 6, characterized in that the temperature during preparation of the concentrated potassium ascorbate solution is kept at a value below 25°C.

8. Process according to one of Claims 1 to 7, characterized in that the dipotassium ascorbate 2-sulphate dihydrate formed during the reaction is isolated from the reaction mixture by addition to the latter of methanol or ethanol, in particular ethanol.

9. Process according to one of Claims 1 to 8, characterized in that the trimethylamine which escapes from the reaction mixture during the reaction is absorbed in a chlorinated organic solvent, in particular in 1,2-dichloroethane, chloroform, carbon tetrachloride or methylene chloride.

10. Process according to Claim 9, characterized in that the tertiary amine-sulphur trioxide complex is recovered from the trimethylamine absorbed or dissolved in the chlorinated organic solvent by adding chlorosulphonic acid to the solution and passing gaseous ammonia into the reaction mixture thus obtained for the purpose of recovery of amine from the amine hydrochloride previously formed as a by-product.

11. Process according to Claim 10, characterized in that ammonia is passed into the reaction mixture mentioned at least until the pH thereof is alkaline.

## Revendications

1. Procédé de préparation du dihydrate de 2-sulfate d'ascorbate de dipotassium de formule 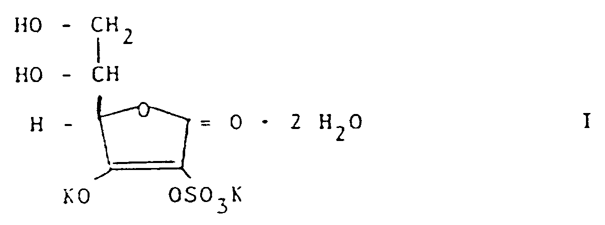 lors duquel on ajoute à une solution d'ascorbate de potassium dans l'eau un complexe de trioxyde de soufre-triméthylamine en excès molaire, on ajuste la valeur pH de ce mélange de réaction à une valeur dans le domaine compris entre 9,5 et 10,5 et on la maintient à cette valeur pendant la durée de la réaction et on isole par addition d'un alcanol le dihydrate de 2-sulfate d'ascorbate de dipotassium qui s'est formé pendant la réaction, caractérisé en ce que l'on ajuste la valeur pH du mélange de réaction grâce à des additions successives d'hydroxyde de potassium, que l'on ajuste la concentration de la solution d'ascorbate de potassium dans l'eau à une valeur dans le domaine compris entre 500 et 750 g/l, que l'excès molaire, dans lequel le complexe de trioxyde de soufre-triméthylamine est ajouté à la solution aqueuse d'ascorbate de potassium, n'est pas de plus de 8 % , et que l'on isole le dihydrate de 2-sulfate d'ascorbate de dipotassium, qui s'est formé pendant la réaction, directement à partir du mélange de réaction grâce à l'addition de l'alcanol à celui-ci.

2. Procédé selon la revendication 1, caractérisé en ce que l'on ajuste la valeur pH à une valeur de 10 et on la maintient à cette valeur pendant la durée de la réaction.

3. Procédé selon l'une quelconque des revendications 1 ou 2, caractérisé en ce que l'excès molaire, dans lequel le complexe de trioxyde de soufre-triméthylamine est ajouté à la solution aqueuse d'ascorbate de potassium, n'est pas de plus de 6 %.

4. Procédé selon l'une quelconque des revendications 1 à 3, caractérisé en ce que l'on ajuste la température du mélange de réaction à une valeur dans le domaine compris entre 57°C et 65°C et que l'on augmente celle-ci, de préférence pendant la durée de la réaction, linéairement de la valeur citée inférieure à la valeur citée supérieure.

5. Procédé selon l'une quelconque des revendications 1 à 4, caractérisé en ce que l'on ajoute simultanément à la solution aqueuse d'ascorbate de potassium la quantité totale de complexe de trioxyde de soufre-triméthylamine.

6. Procédé selon l'une quelconque des revendications 1 à 5, caractérisé en ce que l'on prépare la solution concentrée d'ascorbate de potassium dans l'eau par le mélange d'acide ascorbique-L avec de l'eau et par la neutralisation de ce mélange avec de l'hydroxyde de potassium.

7. Procédé selon la revendication 6, caractérisé en ce que l'on maintient la température lors de la préparation de la solution concentrée d'ascorbate de potassium à une valeur inférieure à 25°C.

8. Procédé selon l'une quelconque des revendications 1 à 7, caractérisé en ce que l'on isole du mélange de réaction le dihydrate de 2-sulfate d'ascorbate de dipotassium, qui s'est formé pendant la réaction, par l'addition au mélange de réaction de méthanol ou d'éthanol, en particulier d'éthanol.

9. Procédé selon l'une quelconque des revendications 1 à 8, caractérisé en ce que la triméthylamine qui s'échappe du mélange de réaction pendant la réaction est absorbée dans un solvant organique chloré, en particulier dans le 1,2-dichloroéthane, le chloroforme, le tétrachlorure de carbone ou le chlorure de méthylène.

10. Procédé selon la revendication 9, caractérisé en ce que l'on extrait de la triméthylamine absorbée, respectivement dissoute dans le solvant organique chloré un complexe de trioxyde de soufre-amine tertiaire, en ce que l'on ajoute de l'acide chlorosulfurique à la solution et en ce que l'on achemine de l'ammoniac sous forme de gaz dans le mélange de réaction ainsi obtenu en vue de la récupération de l'amine en provenance de l'hydrochlorure d'amine obtenu antérieurement en tant que produit secondaire.

11. Procédé selon la revendication 10, caractérisé en ce que l'on achemine de l'ammoniac dans le mélange de réaction cité au moins aussi longtemps qu'il le faut pour que sa valeur pH soit alcaline.
